# EUROPEAN PATENT APPLICATION

(11) **EP 4 682 240 A1**
(43) Date of publication of application: **21.01.2026**
(21) Application number: 24770927.2
(22) Date of filing: 13.03.2024
(51) Int. Cl.: C12N 1/00, B01F 21/00, B01F 23/53, B01F 35/50, B01F 35/81, C12M 1/00

(54) **BIOPROCESS SOLUTION PREPARATION METHOD AND MIXING CONTAINER**

(30) Priority: 15.03.2023 JP 2023041418
(71) Applicant: FUJIFILM Corporation, Tokyo 106-8620 (JP)
(72) Inventor: YAMAMOTO, Takashi, Ashigarakami-gun, Kanagawa 258-8577 (JP); INABA, Tatsuya, Ashigarakami-gun, Kanagawa 258-8577 (JP); HASEGAWA, Masataka, Ashigarakami-gun, Kanagawa 258-8577 (JP)
(74) Representative: Klunker IP Patentanwälte PartG mbB
(86) International application number: PCT/JP2024/009767
(87) International publication number: WO 2024/190822

(57) **Abstract**

A method for preparing a bioprocess solution, in which a bioprocess solution having a set concentration is continuously prepared in a mixing container by continuously supplying a solvent and a solid bioprocess material to the mixing container, the method including storing a first initial solvent, which has a composition closer to the bioprocess solution having the set concentration than the solvent continuously supplied to the mixing container, or a second initial solvent, which has a higher solubility for the solid bioprocess material than the solvent continuously supplied to the mixing container, in the mixing container and starting continuous supply of the solid bioprocess material and the solvent to the mixing container in a state where the first initial solvent or the second initial solvent is stored in the mixing container.

## Description

### BACKGROUND OF THE INVENTION

### 1. Field of the Invention

The technology of the present disclosure relates to a method for preparing a bioprocess solution and a mixing container.

### 2. Description of the Related Art

In a bioprocess in which Chinese hamster ovary cells into which an antibody gene has been incorporated are cultured to obtain an antibody pharmaceutical, various bioprocess solutions are required. WO2021/123248A describes a method of continuously preparing a bioprocess solution having a set concentration by continuously supplying a solvent and a solid bioprocess material to a mixing container and mixing the solvent and the solid bioprocess material in the mixing container. The mixing container is connected to the bioprocess container, and the bioprocess solution is continuously supplied from the mixing container to the bioprocess container. The solvent is, for example, pure water, the solid bioprocess material is, for example, a powdered culture medium, and the bioprocess solution is, for example, a culture medium (culture solution). In addition, the bioprocess container is, for example, a culture tank.

### SUMMARY OF THE INVENTION

The preparation of the bioprocess solution (supply of the solvent and the solid bioprocess material to the mixing container) starts from a state in which the same solvent as the solvent continuously supplied to the mixing container is stored in the mixing container. Therefore, at the initial stage of the preparation, the concentration of the bioprocess solution does not reach the set concentration. The bioprocess solution whose concentration has not reached the set concentration is discarded without being supplied to the bioprocess container. Therefore, the cost of the waste liquid was incurred.

One embodiment according to the technology of the present disclosure provides a method for preparing a bioprocess solution, which can reduce costs by reducing the amount of the bioprocess solution to be discarded, and a mixing container.

A method for preparing a bioprocess solution according to the present disclosure, in which a bioprocess solution having a set concentration is continuously prepared in a mixing container by continuously supplying a solvent and a solid bioprocess material to the mixing container, the method including storing a first initial solvent, which has a composition closer to the bioprocess solution having the set concentration than the solvent continuously supplied to the mixing container, or a second initial solvent, which has a higher solubility for the solid bioprocess material than the solvent continuously supplied to the mixing container, in the mixing container and starting continuous supply of the solvent and the solid bioprocess material to the mixing container in a state where the first initial solvent or the second initial solvent is stored in the mixing container.

It is preferable that the solid bioprocess material includes a poorly soluble material having a relatively slow dissolution rate in the solvent, which remains undissolved and accumulates in the mixing container for a certain period of time after being supplied to the mixing container, and whose solubility increases in a case where a certain amount has accumulated, and the first initial solvent is a liquid in which the poorly soluble material is not dissolved.

It is preferable that the first initial solvent contains a set amount of the poorly soluble material based on the certain amount.

It is preferable that the first initial solvent is produced by using a mixing container.

It is preferable that the first initial solvent is produced by supplying the solvent to a mixing container in which the solid component of the first initial solvent has been previously provided.

It is preferable to use an alkaline liquid as the second initial solvent.

It is preferable to use a liquid having a hydrogen-ion exponent of 8.6 or more and 14 or less as the second initial solvent.

It is preferable to use an acidic liquid as the second initial solvent.

It is preferable to use a liquid having a hydrogen-ion exponent of 0 or more and 5.8 or less as the second initial solvent.

It is preferable that the solid bioprocess material is divided into a plurality of groups according to solubility, the second initial solvent is provided for each of the groups to prepare solutions for each of the groups, and the solutions prepared for each of the groups are mixed to produce the bioprocess solution.

It is preferable that the solvent is purified water, the solid bioprocess material is a powdered culture medium, and the bioprocess solution is a culture medium.

It is preferable that the culture medium is continuously supplied from the mixing container to the culture tank.

It is preferable that the solvent is purified water, the solid bioprocess material is a powder constituting a solid component of a buffer solution, and the bioprocess solution is a buffer solution.

It is preferable that the mixing container is disposable after one use.

It is preferable that the solid bioprocess material is composed of only a single material and the first initial solvent is a liquid in which the one material is mixed.

A mixing container according to the present disclosure, in which a bioprocess solution having a set concentration is continuously prepared by continuously supplying a solvent and a solid bioprocess material, and in which an initial solvent is stored before the continuous supply of the solvent and the solid bioprocess material is started, the initial solvent includes a solid component that is previously provided and has a composition closer to a composition of the bioprocess solution at the set concentration than a composition of the continuously supplied solvent, and the initial solvent is produced by supplying the solvent.

According to the technology of the present disclosure, it is possible to provide a method for preparing a bioprocess solution, which can reduce costs by reducing the amount of the bioprocess solution to be discarded, and a mixing container.

### BRIEF DESCRIPTION OF THE DRAWINGS

Fig. 1 is a diagram showing a culture medium preparation device and a culture tank.
Fig. 2 is a diagram showing a component table of a first initial solvent IS1A.
Fig. 3 is a diagram showing a procedure for producing the first initial solvent IS1A.
Fig. 4 is a flowchart showing a procedure of a culture medium preparation method.
Fig. 5 is a graph showing temporal changes in conductivity in a case where the first initial solvent IS1A is used as an initial solvent and a case where pure water is used as an initial solvent.
Fig. 6 is a diagram showing another example of a procedure for producing the first initial solvent IS1A.
Fig. 7 is a diagram showing another example of a procedure for producing the first initial solvent IS1A.
Fig. 8 is a diagram showing another example of a procedure for producing the first initial solvent IS1A.
Fig. 9 is a diagram showing a component table of a first initial solvent IS1B.
Fig. 10 is a graph showing temporal changes in conductivity in a case where the first initial solvent IS1B is used as an initial solvent and a case where pure water is used as an initial solvent.
Fig. 11 is a diagram showing a second initial solvent IS2A.
Fig. 12 is a graph showing temporal changes in conductivity in a case where the second initial solvent IS2A is used as an initial solvent and a case where pure water is used as an initial solvent.
Fig. 13 is a diagram showing a second initial solvent IS2B.
Fig. 14 is a graph showing temporal changes in conductivity in a case where the second initial solvent IS2B is used as an initial solvent and a case where pure water is used as an initial solvent.
Fig. 15 is a diagram showing a fifth embodiment in which a second initial solvent is provided for each of a plurality of groups obtained by dividing a powdered culture medium according to solubility, to prepare a solution for each group, and the solutions prepared for each group are mixed to produce a culture medium.
Fig. 16 is a table summarizing Examples and Comparative Examples.
Fig. 17 is a diagram showing another example of the bioprocess solution.
Fig. 18 is a diagram showing a solid process material composed of only a single material and a first initial solvent in which the one material is mixed.

### DESCRIPTION OF THE PREFERRED EMBODIMENTS

### [First embodiment]

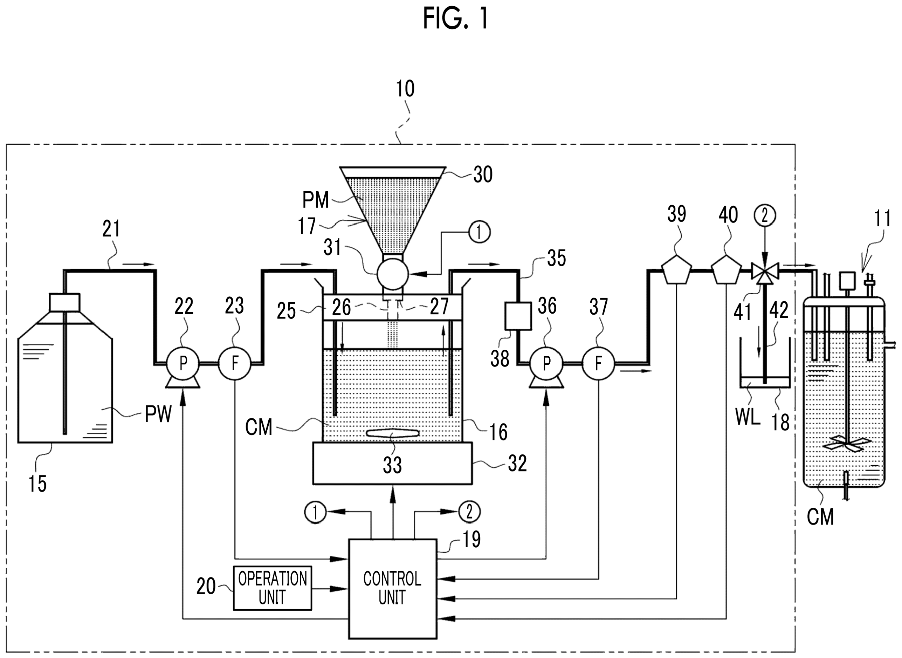

As shown in Fig. 1 as an example, the culture medium preparation device 10 prepares a culture medium CM. The culture medium preparation device 10 is connected to the culture tank 11, and continuously supplies the prepared culture medium CM to the culture tank 11. The culture medium CM is an example of a "bioprocess solution" according to the technology of the present disclosure. It is noted that in Fig. 1, an example in which the culture medium preparation device 10 is directly connected to the culture tank 11 has been described, but the present invention is not limited thereto. A tank for temporarily storing the culture medium CM may be provided between the culture medium preparation device 10 and the culture tank 11.

For example, Chinese hamster ovary cells into which an antibody gene has been incorporated are seeded in the culture tank 11. In the culture tank 11, Chinese hamster ovary cells are cultured in the culture medium CM. As described above, since the culture medium CM is continuously supplied to the culture tank 11 from the culture medium preparation device 10, the culture of the Chinese hamster ovary cells performed in the culture tank 11 is perfusion culture. The culture tank 11 is connected to a purification device (not shown). The purification device purifies an antibody produced by Chinese hamster ovary cells in the culture process, and yields the antibody as an active pharmaceutical ingredient of an antibody pharmaceutical.

The culture medium preparation device 10 includes a pure water storage tank 15, a mixing container 16, a powdered culture medium supply unit 17, a waste liquid recovery tank 18, a control unit 19, an operation unit 20, and the like.

The pure water storage tank 15 stores pure water PW. The pure water PW is an example of a "solvent", a "solvent continuously supplied to a mixing container", and "purified water" according to the technology of the present disclosure.

One end of the pure water supply line 21 is connected to the pure water storage tank 15. The other end of the pure water supply line 21 is connected to the mixing container 16. A pure water supply pump 22 and a pure water flowmeter 23 are provided in the pure water supply line 21. The pure water supply pump 22 is driven and controlled by the control unit 19. In a case where the pure water supply pump 22 is driven, the pure water PW in the pure water storage tank 15 is supplied to the mixing container 16 through the pure water supply line 21. The pure water flowmeter 23 is disposed downstream of the pure water supply pump 22, and measures a flow rate of the pure water PW passing through the pure water supply line 21. The pure water flowmeter 23 outputs the measured flow rate to the control unit 19. The control unit 19 controls the driving of the pure water supply pump 22 such that the flow rate of the pure water flowmeter 23 is a set amount.

The mixing container 16 has, for example, a wide, straight-sided cylindrical shape having an open upper portion, and is a container for mixing the pure water PW and the powdered culture medium PM to produce a culture medium CM having a set concentration. The upper portion of the mixing container 16 is sealed with a lid 25. A through hole 26 is formed in a center portion of the lid 25. A discharge port 27 for the powdered culture medium PM of the powdered culture medium supply unit 17 is aseptically connected to an upper end part of the through hole 26. As a result, the mixing container 16 and the powdered culture medium supply unit 17 are aseptically connected. The powdered culture medium PM discharged from the discharge port 27 is supplied to the mixing container 16 through the through hole 26. The powdered culture medium PM is an example of a "solid bioprocess material" according to the technology of the present disclosure.

The powdered culture medium supply unit 17 is composed of a funnel-shaped hopper 30 that stores the powdered culture medium PM and a feeder 31 that continuously discharges the powdered culture medium PM in the hopper 30 from the discharge port 27. The feeder 31 is attached to a lower, reduced-diameter portion of the hopper 30. The feeder 31 is driven and controlled by the control unit 19, and continuously discharges the set amount of the powdered culture medium PM from the discharge port 27. The feeder 31 is, for example, a screw feeder that continuously discharges a set amount of the powdered culture medium PM from the discharge port 27 by rotationally driving a helical screw by a motor under the control of the control unit 19. Although not shown, a stirrer for crushing rat holes generated by long-term use is introduced into the hopper 30. The inner wall of the hopper 30 may be subjected to a sliding treatment for improving the discharge properties of the powdered culture medium PM. In addition, the feeder 31 is not limited to the screw feeder, and may be a table feeder, a rotary feeder, a belt feeder, or the like.

The mixing container 16 is installed on the stirrer 32. A stirring bar 33 is put in the mixing container 16. The stirring bar 33 has an elongated cocoon-like shape in which both end portions are rounded and a central portion is slightly swollen. A magnet is built in the stirring bar 33. The stirrer 32 is driven and controlled by the control unit 19. The stirrer 32 generates a magnetic force for rotating the stirring bar 33. The stirrer 32 rotates the stirring bar 33 at a set rotation speed to mix the pure water PW and the powdered culture medium PM to obtain a culture medium CM.

One end of the culture medium supply line 35 is connected to the mixing container 16. The other end of the culture medium supply line 35 is connected to the culture tank 11. A culture medium supply pump 36 and a culture medium flowmeter 37 are provided in the culture medium supply line 35. The culture medium supply pump 36 is driven and controlled by the control unit 19. In a case where the culture medium supply pump 36 is driven, the culture medium CM in the mixing container 16 is supplied to the culture tank 11 through the culture medium supply line 35. The culture medium flowmeter 37 is disposed downstream of the culture medium supply pump 36, and measures the flow rate of the culture medium CM passing through the culture medium supply line 35. The culture medium flowmeter 37 outputs the measured flow rate to the control unit 19. The control unit 19 controls the driving of the culture medium supply pump 36 such that the flow rate of the culture medium flowmeter 37 is a set amount.

The flow rate of the pure water PW and the flow rate of the culture medium CM are set to values such that the amount of liquid in the mixing container 16 does not change from the beginning to the end. More specifically, the flow rate of the culture medium CM is set according to the combined amount of the flow rate of the pure water PW and the supply amount of the powdered culture medium PM. In practice, the weight of the mixing container 16 is measured with a weighing scale, and the flow rate of the pure water PW and the flow rate of the culture medium CM are set such that the measured weight is constant.

A filter 38 is disposed upstream of the culture medium supply pump 36 of the culture medium supply line 35. The filter 38 removes unnecessary substances in the culture medium CM. The unnecessary substance is, for example, an undissolved powdered culture medium PM, and the like.

A conductivity meter 39 and a hydrogen-ion exponent meter 40 are disposed downstream of the culture medium flowmeter 37 in the culture medium supply line 35. In addition, a waste liquid line 42 is connected to the downstream of the hydrogen-ion exponent meter 40 of the culture medium supply line 35 via a three-way valve 41. The three-way valve 41 is driven and controlled by the control unit 19. The waste liquid recovery tank 18 is connected to the waste liquid line 42. The waste liquid recovery tank 18 recovers the waste liquid WL which is the culture medium CM not having the set concentration or the culture medium CM having an abnormal value of the hydrogen-ion exponent.

The conductivity meter 39 measures the conductivity (unit: mS/cm) of the culture medium CM that passes through the culture medium supply line 35. The conductivity meter 39 outputs the measured conductivity to the control unit 19. The control unit 19 determines whether or not the culture medium CM has the set concentration on the basis of the conductivity from the conductivity meter 39. In a case where it is determined that the culture medium CM is not at the set concentration, the control unit 19 sets the flow passage of the three-way valve 41 to the waste liquid line 42 side, and discharges the waste liquid WL, which is the culture medium CM not having the set concentration, to the waste liquid recovery tank 18. On the other hand, in a case where it is determined that the culture medium CM has the set concentration, the control unit 19 sets the flow passage of the three-way valve 41 to the culture tank 11 side, and introduces the culture medium CM having the set concentration into the culture tank 11. In this way, only the culture medium CM having the set concentration is supplied to the culture tank 11. Accordingly, it is possible to prevent a problem such as the supply of the culture medium CM not having the set concentration to the culture tank 11 and the failure of the culture of the Chinese hamster ovary cells.

The hydrogen-ion exponent meter 40 measures the hydrogen-ion exponent of the culture medium CM that passes through the culture medium supply line 35. The hydrogen-ion exponent meter 40 outputs the measured hydrogen-ion exponent to the control unit 19. In a case where the hydrogen-ion exponent from the hydrogen-ion exponent meter 40 is an abnormal value, the control unit 19 sets the flow passage of the three-way valve 41 to the waste liquid line 42 side, and discharges the waste liquid WL, which is the culture medium CM having an abnormal value of the hydrogen-ion exponent, to the waste liquid recovery tank 18.

The control unit 19 includes, for example, a processor such as a central processing unit (CPU), a memory, and a storage, and comprehensively controls driving of each unit of the culture medium preparation device 10. The processor of the control unit 19 may be a programmable logic device (PLD) that is a processor of which a circuit configuration can be changed after manufacture, such as a field programmable gate array (FPGA), a dedicated electric circuit that is a processor having a circuit configuration dedicatedly designed to execute specific processing, such as an application specific integrated circuit (ASIC), or the like.

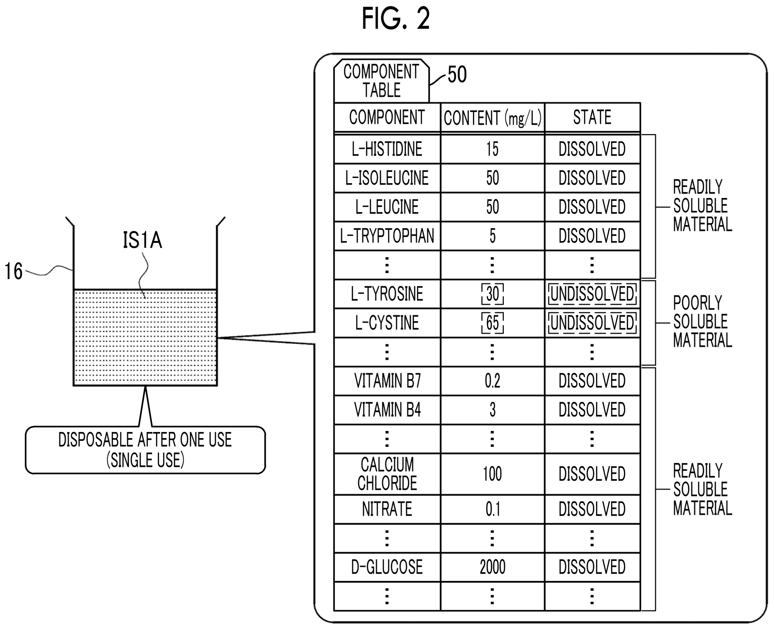

The operation unit 20 is, for example, a touch panel, and receives various operation instructions from an operator of the culture medium preparation device 10. The various operation instructions include a production instruction for the first initial solvent IS1A (see Fig. 2), a start instruction for continuous supply of the pure water PW and the powdered culture medium PM to the mixing container 16, a forced stop instruction for the supply of the culture medium CM from the mixing container 16 to the culture tank 11, or the like.

The pure water storage tank 15, the mixing container 16, the hopper 30 and the feeder 31 of the powdered culture medium supply unit 17, and the waste liquid recovery tank 18 are disposable after one use, that is, single-use (see Fig. 2 for the mixing container 16). In addition, the pure water supply line 21, the culture medium supply line 35, the filter 38, the three-way valve 41, and the waste liquid line 42 are also single-use.

All flow passages from the pure water storage tank 15 to the culture tank 11 through the pure water supply line 21, the mixing container 16, and the culture medium supply line 35 are aseptically connected. In addition, the culture medium supply line 35, the three-way valve 41, and the waste liquid line 42 are also aseptically connected.

As shown in Fig. 2 as an example, the culture medium preparation device 10 uses the first initial solvent IS1A. The culture medium preparation device 10 starts the continuous supply of the pure water PW and the powdered culture medium PM to the mixing container 16 in a state where the set amount of the first initial solvent IS1A is stored in the mixing container 16. The initial solvent such as the first initial solvent IS1A refers to a liquid in the mixing container 16 before the continuous supply of the pure water PW and the powdered culture medium PM to the mixing container 16 is started. On the other hand, the culture medium CM refers to a liquid in the mixing container 16 after the continuous supply of the pure water PW and the powdered culture medium PM to the mixing container 16 is started.

As shown in the component table 50, the first initial solvent IS1A contains various materials (compounds) required for the culture medium CM, such as an amino acid such as L-histidine, vitamins such as vitamin B7, inorganic salts such as calcium chloride, and saccharides such as D-glucose. Therefore, the composition of the first initial solvent IS1A is closer to the that of the culture medium CM having a set concentration than that of the pure water PW continuously supplied to the mixing container 16. It can be said that, in a liquid in which any component of the culture medium CM is present even slightly, the composition is closer to that of the culture medium CM having the set concentration than that of the pure water PW.

The materials contained in the first initial solvent IS1A are classified into an readily soluble material and a poorly soluble material. The readily soluble material is a material having a relatively high dissolution rate in pure water PW. The readily soluble material is present in a state of being dissolved in the first initial solvent IS1A. On the other hand, the poorly soluble material is a material having a relatively slow dissolution rate in pure water PW. The poorly soluble material accumulates in the mixing container 16 for a certain period of time without being dissolved after being supplied from the powdered culture medium supply unit 17 to the mixing container 16, and the solubility increases in a case where a certain amount of the poorly soluble material is accumulated. The poorly soluble material is present in an undissolved state in the first initial solvent IS1A. The readily soluble materials are L-histidine, L-tryptophan, vitamin B7, calcium chloride, D-glucose, and the like. The poorly soluble materials are L-tyrosine, L-cystine, and the like. The above-described certain time and the above-described certain amount are determined by the type of the poorly soluble material, the amount of pure water, the temperature of pure water, and the like.

The content of the poorly soluble materials such as 30 mg/L of L-tyrosine and 65 mg/L of L-cystine is a set amount based on the above-described certain amount. The set amount is, for example, the same amount as a certain amount. The term "the same" mentioned here refers to not only "completely the same" but also "the same" in the sense of including an error that is generally allowed in a technical field to which the technique of the present disclosure belongs and that does not contradict the gist of the technique of the present disclosure. The content of the poorly soluble material may be more than a certain amount or may be conversely less than the certain amount.

Similar to the first initial solvent IS1A, the powdered culture medium PM supplied to the mixing container 16 by the powdered culture medium supply unit 17 also contains the readily soluble material and the poorly soluble material in a mixed state.

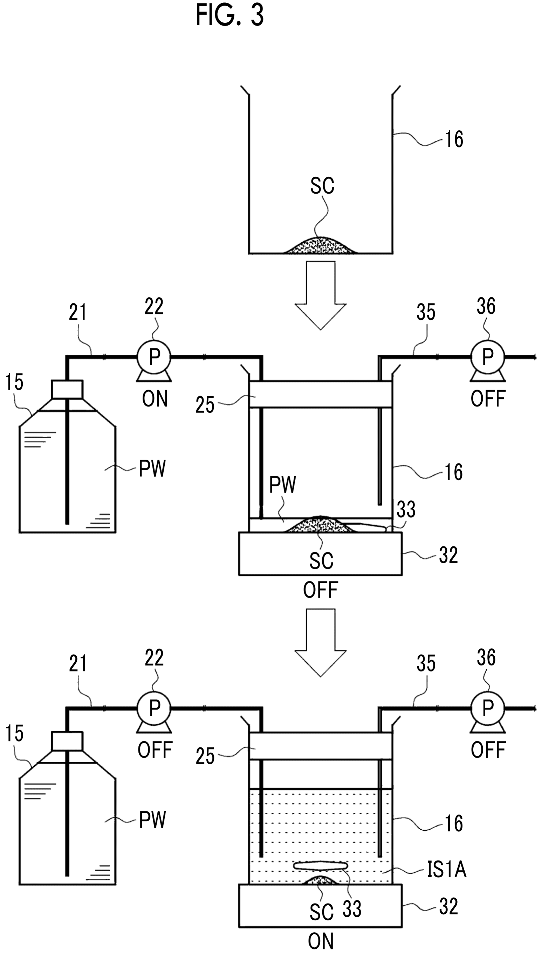

The first initial solvent IS1A is produced by the procedure shown in Fig. 3 as an example. First, as shown in the upper panel, a mixing container 16 in which the solid component SC has been previously provided is prepared. The solid component SC includes all the components of the first initial solvent IS1A shown in the component table 50 of Fig. 2. The solid component SC is in a powdery or blocky form and is allowed to stand on a bottom surface of the mixing container 16. Here, the term "previously provided" does not mean that the operator of the culture medium preparation device 10 prepares the solid component SC, but means that the manufacturer of the mixing container 16 prepares the solid component SC and sells the mixing container 16 containing the solid component SC. In analogy to an instant cup noodle, the mixing container 16 corresponds to a container of cup noodles, and the solid components SC corresponds to noodles, toppings (additional ingredients), and soup base.

As shown in the middle panel, the operator sets the mixing container 16 in the culture medium preparation device 10. Thereafter, the operator inputs the instruction to produce the first initial solvent IS1A via the operation unit 20. In response to the production instruction, the control unit 19 supplies the pure water PW to the mixing container 16 by driving only the pure water supply pump 22 while the driving of the culture medium supply pump 36 is stopped.

In a case where the supply amount of the pure water PW has reached the set amount, as shown in the lower panel, the control unit 19 terminates the driving of the pure water supply pump 22 to stop the supply of the pure water PW to the mixing container 16. The determination of whether or not the supply amount of the pure water PW has reached the set amount is performed based on the flow rate of the pure water PW measured by the pure water flowmeter 23. Alternatively, it may be performed based on the detection result of the liquid surface height of the liquid in the mixing container 16.

After the supply of the pure water PW to the mixing container 16 is stopped, the control unit 19 drives the stirrer 32 to stir the pure water PW and the solid component SC for a set time. As the set time, a time in which the readily soluble material is dissolved but the poorly soluble material is not dissolved and the set amount thereof is accumulated is set. In this way, the first initial solvent IS1A is produced. In this way, the first initial solvent IS1A is produced by using the mixing container 16. It is noted that in a case where the readily soluble material is dissolved only by supplying pure water PW without stirring, the stirring step may be omitted.

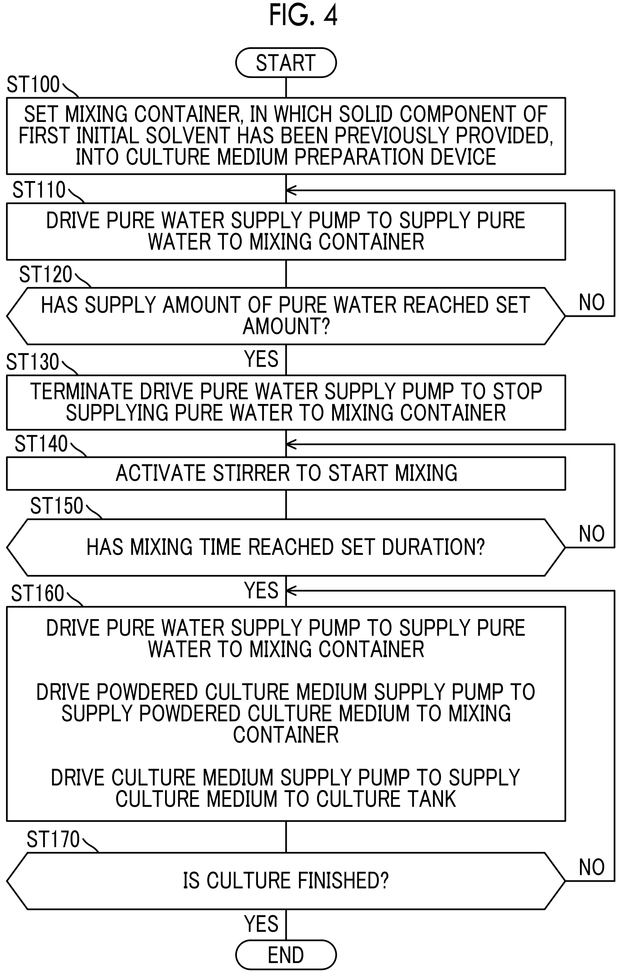

Next, an operation of the configuration described above will be described with reference to the flowchart shown in Fig. 4 as an example. First, as shown in Fig. 3, a mixing container 16 in which the solid components SC of the first initial solvent IS1A has been previously provided is set in the culture medium preparation device 10 by an operator (Step ST100). Then, the operator gives an instruction to produce the first initial solvent IS1A through the operation unit 20. As a result, the pure water supply pump 22 is driven under the control of the control unit 19 to supply the pure water PW to the mixing container 16 (Step ST110). The supply of the pure water PW to the mixing container 16 is continued as long as the supply amount of the pure water PW has not reached the set amount (NO in Step ST120).

In a case where the supply amount of the pure water PW has reached the set amount (YES in Step ST120), the driving of the pure water supply pump 22 is terminated under the control of the control unit 19, and the supply of the pure water PW to the mixing container 16 is stopped (Step ST130). Then, the stirrer 32 is driven and the pure water PW and the solid component SC are stirred under the control of the control unit 19 (Step ST140). The stirring is continued as long as the stirring time has not reached the set time (NO in Step ST150).

In a case where the stirring time has reached the set time (YES in Step ST150), the pure water supply pump 22 is driven under the control of the control unit 19 to supply the pure water PW to the mixing container 16 (Step ST160). At the same time, the powdered culture medium supply unit 17 is driven under the control of the control unit 19 to supply the powdered culture medium PM to the mixing container 16 (Step ST160). That is, the continuous supply of the pure water PW and the powdered culture medium PM to the mixing container 16 is started in a state where the first initial solvent IS1A is stored in the mixing container 16.

In addition, the culture medium supply pump 36 is driven under the control of the control unit 19 to supply the culture medium CM to the culture tank 11 (Step ST160). The continuous supply of the pure water PW and the powdered culture medium PM to the mixing container 16 and the continuous supply of the culture medium CM to the culture tank 11 are continued as long as the culturing in the culture tank 11 has not completed (NO in Step ST170). In a case where the culturing in the culture tank 11 is ended (YES in Step ST170), the driving of the pure water supply pump 22, the powdered culture medium supply unit 17, and the culture medium supply pump 36 is terminated under the control of the control unit 19, and the supply of the pure water PW and the powdered culture medium PM to the mixing container 16 and the supply of the culture medium CM to the culture tank 11 are stopped.

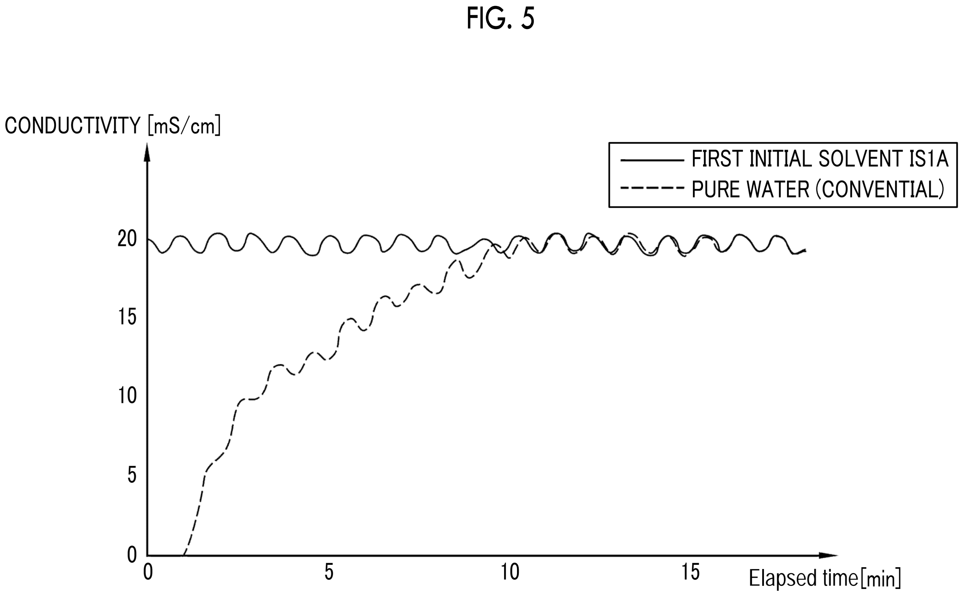

Fig. 5 is a graph showing temporal changes in conductivity of the culture medium CM after the start of the supply of the pure water PW and the powdered culture medium PM to the mixing container 16. The solid line indicates a case where the first initial solvent IS1A is used as the initial solvent, and the broken line indicates a case where pure water is used as the initial solvent as in the related art. In a case where pure water is used as the initial solvent, it takes about 9 minutes until the conductivity reaches the set value (the concentration reaches the set concentration). Therefore, all the culture medium CM produced up to that time is discarded as the waste liquid WL into the waste liquid recovery tank 18 through the waste liquid line 42. On the other hand, in a case where the first initial solvent IS1A is used as the initial solvent, the conductivity immediately reaches the set value (the concentration reaches the set concentration) after the start of the supply of the pure water PW and the powdered culture medium PM to the mixing container 16. Therefore, there is almost no culture medium CM to be discarded into the waste liquid recovery tank 18 as the waste liquid WL, or the amount thereof is extremely small. It is noted that in any case of using the first initial solvent IS1A or using pure water, a periodic variation in conductivity is observed, which is due to the rotation of the screw of the feeder 31 of the powdered culture medium supply unit 17. The periodic variation in conductivity does not affect the determination of whether or not the conductivity has reached the set value (the concentration has reached the set concentration).

As described above, in the technique of the present disclosure, the first initial solvent IS1A having a composition closer to that of the culture medium CM having a set concentration than that of the pure water PW continuously supplied to the mixing container 16 is stored in the mixing container 16. Then, in a state where the first initial solvent IS1A is stored in the mixing container 16, the continuous supply of the pure water PW and the powdered culture medium PM to the mixing container 16 is started. Therefore, it is possible to reduce the amount of the culture medium CM to be discarded, and it is possible to reduce the cost.

The powdered culture medium PM contains a poorly soluble material having a relatively slow dissolution rate in pure water PW. The poorly soluble material accumulates in the mixing container 16 without being dissolved for a certain period of time after being supplied to the mixing container 16, and the solubility increases in a case where a certain amount of the poorly soluble material is accumulated. The first initial solvent IS1A is a liquid in which a poorly soluble material is undissolved. The first initial solvent IS1A contains a poorly soluble material in a set amount based on a certain amount. Therefore, after the start of the supply of the pure water PW and the powdered culture medium PM to the mixing container 16, the poorly soluble material can be dissolved without waiting for the poorly soluble material to accumulate to a certain amount in the mixing container 16 after the start of the supply of the powdered culture medium PM to the mixing container 16. The culture medium CM can be set to the set concentration in a shorter time as compared with a case where the first initial solvent IS 1A does not contain an undissolved poorly soluble material. As a result, the amount of the culture medium CM to be discarded can be further reduced. It is noted that in the above-described example, a case where the powdered culture medium PM as the solid bioprocess material includes both the readily soluble material and the poorly soluble material in a mixed state is shown, but the present invention is not limited thereto. The solid bioprocess material may be composed of only a readily soluble material or may be composed of only a poorly soluble material.

As shown in Fig. 3, the first initial solvent ISIA is produced by using the mixing container 16. Therefore, it is not necessary to prepare another container for producing the first initial solvent IS1A or to transfer the first initial solvent IS1A from another container to the mixing container 16. Therefore, it is possible to reduce the workload of the operator.

In addition, the first initial solvent IS1A is produced by using a mixing container 16 in which the solid component SC of the first initial solvent IS1A has been previously provided and supplying pure water PW to the mixing container 16. Therefore, the first initial solvent IS1A can be easily produced, and the workload of the operator can be further reduced.

As shown in Fig. 1, the solvent is pure water PW, the solid bioprocess material is a powdered culture medium PM, and the bioprocess solution is a culture medium CM. Therefore, the culture medium CM, which is essential for culturing, can be prepared at a low cost.

In addition, the culture medium CM is continuously supplied from the mixing container 16 to the culture tank 11. Therefore, it is possible to employ perfusion culture, and it is possible to perform a relatively long-term culture close to a physiological environment.

As shown in Fig. 2, the mixing container 16 is disposable after one use. Therefore, it is possible to suppress contamination due to repeated use.

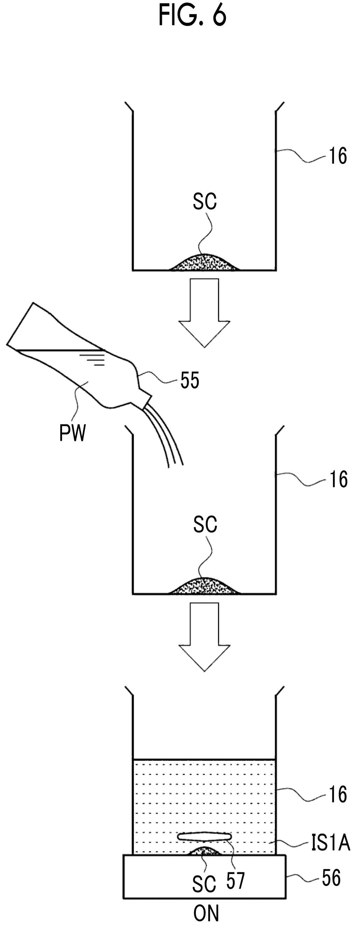

The method of producing the first initial solvent IS1A is not limited to the method shown in Fig. 3. As an example, the methods shown in Figs. 6 to 8 may be used.

The example shown in Fig. 6 is the same as the method shown in Fig. 3 up to the point where, as shown in the upper panel, a mixing container 16 in which the solid component SC of the first initial solvent IS1A has been previously provided. However, in the example shown in Fig. 6, as shown in the middle panel, the operator supplies the set amount of the pure water PW from the bottle 55 to the mixing container 16 at a place different from the culture medium preparation device 10 without setting the mixing container 16 in the culture medium preparation device 10. Thereafter, as shown in the lower panel, the operator stirs the pure water PW and the solid component SC for a set time using a stirrer 56 and a stirring bar 57 different from the stirrer 32 and the stirring bar 33 of the culture medium preparation device 10. After stirring for the set time, the operator sets the mixing container 16 in the culture medium preparation device 10.

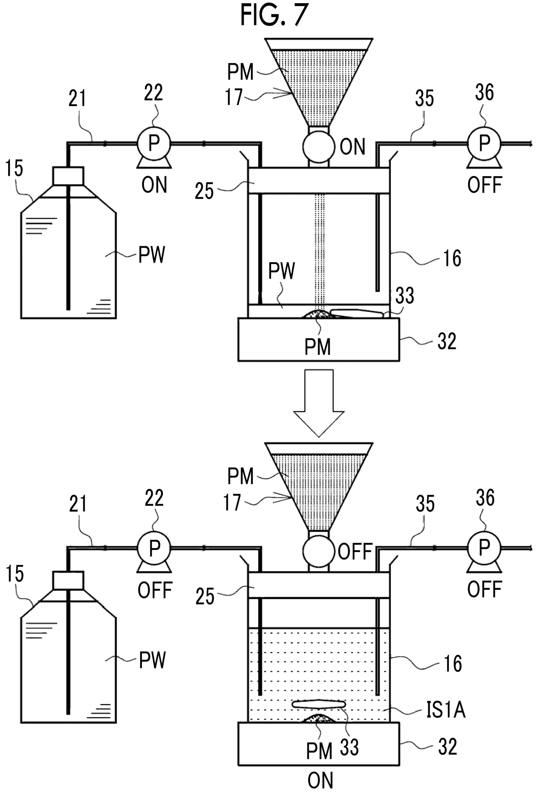

In the example shown in Fig. 7, as shown in the upper panel, the operator prepares a mixing container 16 which is empty and does not contain the solid component SC, and sets the mixing container 16 in the culture medium preparation device 10. Thereafter, the operator inputs the instruction to produce the first initial solvent IS1A via the operation unit 20. In response to the production instruction, the control unit 19 drives the pure water supply pump 22 to supply the pure water PW to the mixing container 16. In addition, the control unit 19 drives the powdered culture medium supply unit 17 to supply the powdered culture medium PM to the mixing container 16.

In a case where the supply amounts of the pure water PW and the powdered culture medium PM reach the set amounts, the control unit 19 terminates the driving of the pure water supply pump 22 to stop the supply of the pure water PW to the mixing container 16, as shown in the lower panel. In addition, the control unit 19 terminates the driving of the powdered culture medium supply unit 17 to stop the supply of the powdered culture medium PM to the mixing container 16. After the supply of the pure water PW and the powdered culture medium PM to the mixing container 16 is stopped, the control unit 19 drives the stirrer 32 to stir the pure water PW and the powdered culture medium PM for a set time.

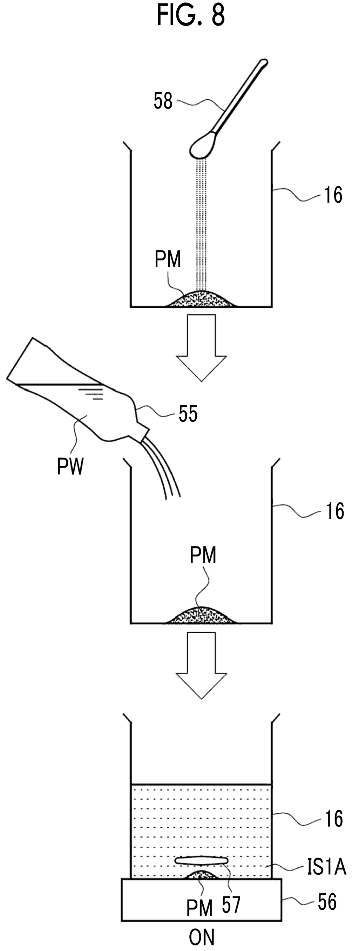

The example shown in Fig. 8 is the same as the method shown in Fig. 7 up to the point where, as shown in the upper panel, a mixing container 16 which is empty and does not contain the solid component SC. Provided that in the example shown in Fig. 8, the operator supplies the set amount of the powdered culture medium PM to the mixing container 16 using the spoon 58 or the like at a place different from the culture medium preparation device 10 without setting the mixing container 16 in the culture medium preparation device 10.

As shown in the middle panel, the operator supplies the set amount of pure water PW from the bottle 55 to the mixing container 16, as in the case of Fig. 6. Thereafter, as shown in the lower panel, the operator stirs the pure water PW and the powdered culture medium PM for a set time using the stirrer 56 and the stirring bar 57, as in the case of Fig. 6. After stirring for the set time, the operator sets the mixing container 16 in the culture medium preparation device 10.

As described above, there are various methods for producing the first initial solvent IS1A. Provided that the method shown in Fig. 3 can most reduce the workload of the operator.

### [Second embodiment]

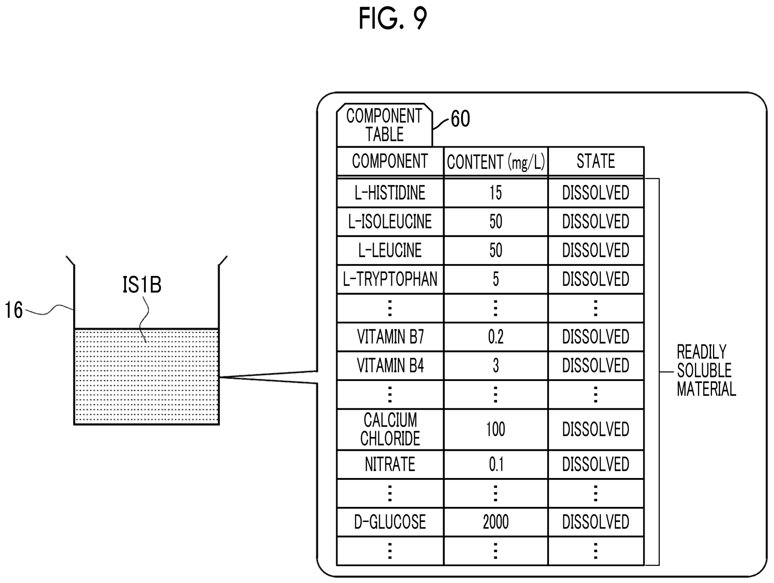

As shown in Fig. 9 as an example, according to the component list 60, the first initial solvent IS1B of the second embodiment is a liquid in which only the readily soluble material is dissolved, and does not include the undissolved poorly soluble material. It is noted that the first initial solvent IS1B may be produced by the method shown in Fig. 3 or Fig. 6 using the mixing container 16 in which the solid component SC has been previously provided, or may be produced by the method shown in Fig. 7 or Fig. 8 using an mixing container 16 which is empty and does not contain the solid component SC.

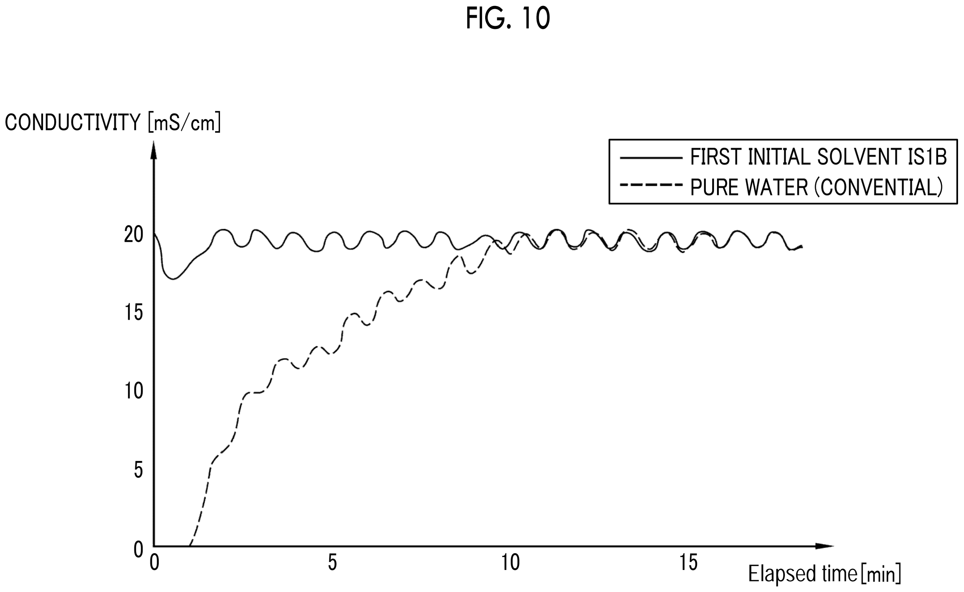

Fig. 10 is a graph showing temporal changes in conductivity of the culture medium CM in the second embodiment. The solid line indicates a case where the first initial solvent IS1B is used as the initial solvent, and the broken line indicates a case where pure water is used as the initial solvent as in Fig. 5. In a case where the first initial solvent IS1B is used as the initial solvent, the conductivity reaches the set value (the concentration reaches the set concentration) in 1 minute after the start of the supply of the pure water PW and the powdered culture medium PM to the mixing container 16. Therefore, the culture medium CM to be discarded in the waste liquid recovery tank 18 as the waste liquid WL is significantly reduced to about 1/9 as compared with a case where pure water is used as the initial solvent. In this way, even with the first initial solvent IS1B that does not contain the undissolved poorly soluble material, the amount of the culture medium CM to be discarded can be reduced, and the cost can be reduced. It is considered that the reason why the conductivity is reduced immediately after the supply of the pure water PW and the powdered culture medium PM to the mixing container 16 is started and the time taken for the conductivity to reach the set value is longer than in a case where the first initial solvent IS1A is used as the initial solvent is that the undissolved poorly soluble material is not contained.

### [Third embodiment]

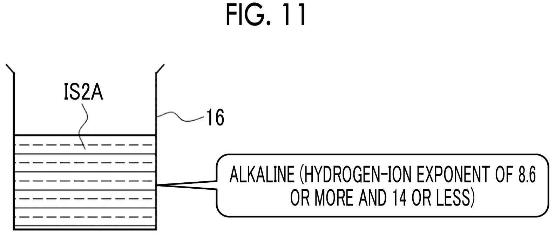

As shown in Fig. 11 as an example, in the third embodiment, the second initial solvent IS2A is used as the initial solvent. The second initial solvent IS2A has a higher solubility for the powdered culture medium PM than the pure water PW continuously supplied to the mixing container 16. Specifically, the second initial solvent IS2A is an alkaline liquid having a hydrogen-ion exponent of 8.6 or more and 14 or less. The second initial solvent IS2A is, for example, a sodium hydroxide solution. The operator appropriately dilutes a standard solution of a commercially available 1 mol/L sodium hydroxide solution to produce the second initial solvent IS2A. It is noted that the second initial solvent IS2A may be produced by using the mixing container 16, or may be produced by using a container different from the mixing container 16 and transferred to the mixing container 16.

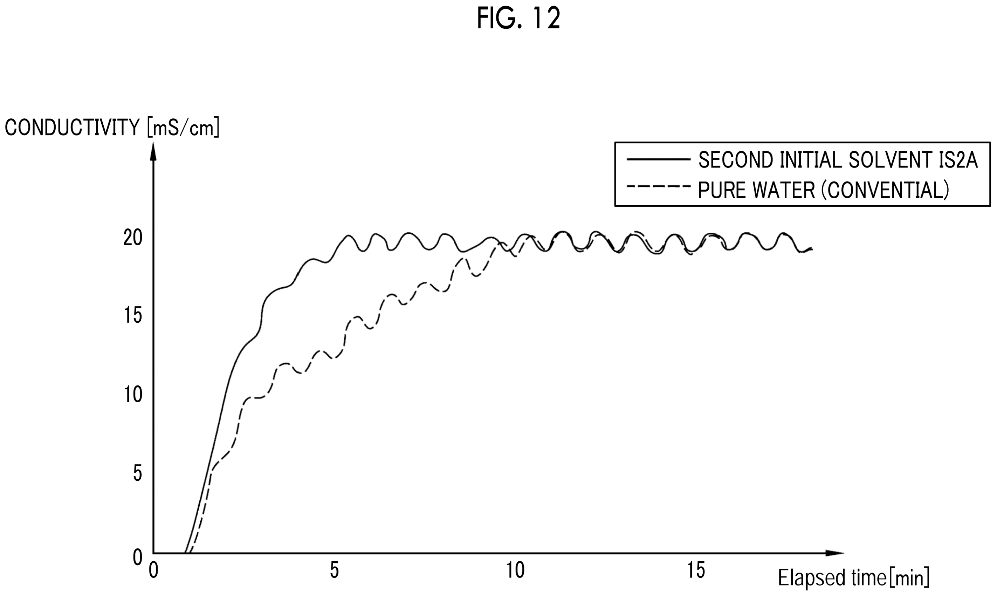

Fig. 12 is a graph showing temporal changes in conductivity of the culture medium CM in the third embodiment. The solid line indicates a case where the second initial solvent IS2A is used as the initial solvent, and the broken line indicates a case where pure water is used as the initial solvent as in Fig. 5 or the like. In a case where the second initial solvent IS2A is used as the initial solvent, the conductivity reaches the set value (the concentration reaches the set concentration) in 4 minute after the start of the supply of the pure water PW and the powdered culture medium PM to the mixing container 16. Therefore, the culture medium CM to be discarded in the waste liquid recovery tank 18 as the waste liquid WL is reduced to about 4/9 as compared with a case where pure water is used as the initial solvent. In this way, even with the first initial solvent IS2A that is an alkaline liquid having a hydrogen-ion exponent of 8.6 or more and 14 or less, the amount of the culture medium CM to be discarded can be reduced, and the cost can be reduced.

### [Fourth embodiment]

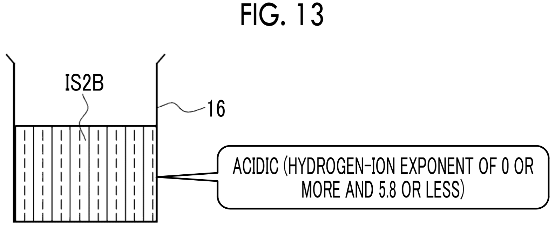

As shown in Fig. 13 as an example, in the fourth embodiment, the second initial solvent IS2B is used as the initial solvent. The second initial solvent IS2B has a higher solubility for the powdered culture medium PM than the pure water PW continuously supplied to the mixing container 16, similarly to the initial solvent IS2A. Specifically, the second initial solvent IS2B is an acidic liquid having a hydrogen-ion exponent of 0 or more and 5.8 or less. The second initial solvent IS2B is, for example, a hydrochloric acid solution. The operator appropriately dilutes a standard solution of a commercially available 1 mol/L hydrochloric acid solution to produce the second initial solvent IS2B. It is noted that the second initial solvent IS2B may be produced by using the mixing container 16, or may be produced by using a container different from the mixing container 16 and transferred to the mixing container 16, similarly to the initial solvent IS2A.

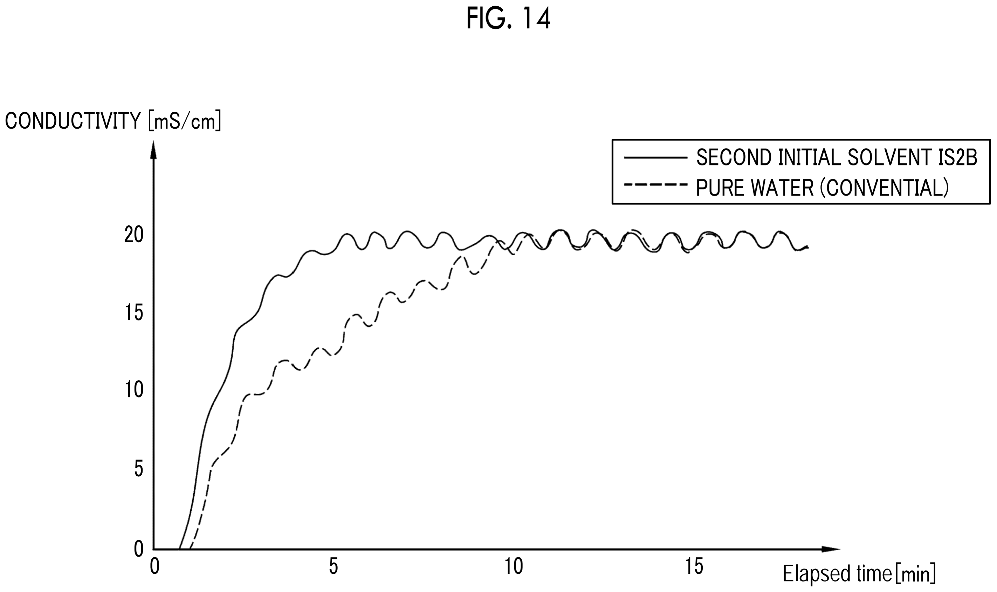

Fig. 14 is a graph showing temporal changes in conductivity of the culture medium CM in the fourth embodiment. The solid line indicates a case where the second initial solvent IS2B is used as the initial solvent, and the broken line indicates a case where pure water is used as the initial solvent as in Fig. 5 or the like. In a case where the second initial solvent IS2B is used as the initial solvent, the conductivity reaches the set value (the concentration reaches the set concentration) in 5 minutes after the start of the supply of the pure water PW and the powdered culture medium PM to the mixing container 16. Therefore, the culture medium CM to be discarded in the waste liquid recovery tank 18 as the waste liquid WL is reduced to about 5/9 as compared with a case where pure water is used as the initial solvent. In this way, even with the first initial solvent IS2B that is an acidic liquid having a hydrogen-ion exponent of 0 or more and 5.8 or less, the amount of the culture medium CM to be discarded can be reduced, and the cost can be reduced. It is considered that the reason why the time taken for the conductivity to reach the set value is longer than in a case where the second initial solvent IS2A is used as the initial solvent is that the content of the material having high solubility in the second initial solvent IS2B is lower than that of the material having high solubility in the second initial solvent IS2A in the powdered culture medium PM.

### [Fifth embodiment]

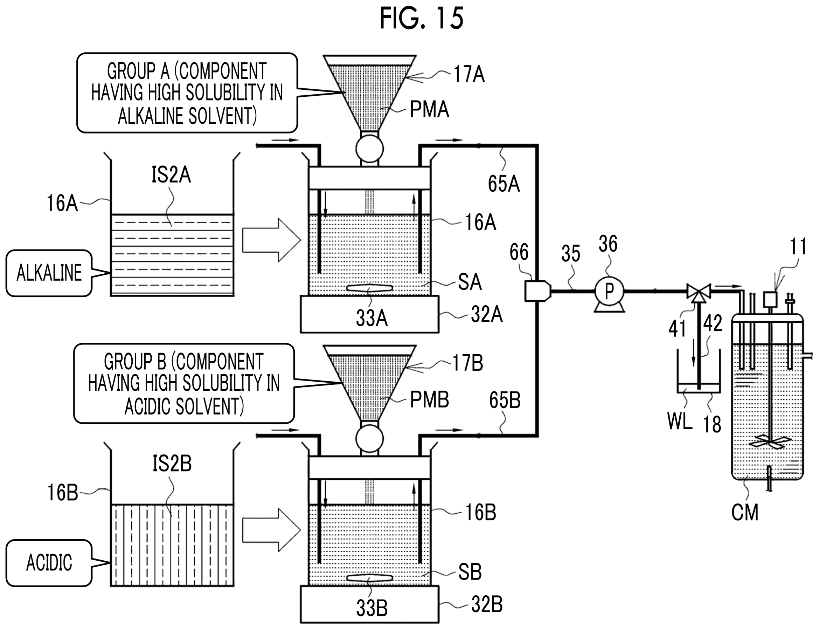

As shown in Fig. 15 as an example, in the fifth embodiment, the second initial solvent IS2A of the third embodiment is prepared in a mixing container 16A, and the second initial solvent IS2B of the fourth embodiment is prepared in a mixing container 16B. In addition, a powdered culture medium supply unit 17A for the mixing container 16A, a stirrer 32A, and a stirring bar 33A, and a powdered culture medium supply unit 17B for the mixing container 16B, a stirrer 32B, and a stirring bar 33B are prepared. Then, the powdered culture medium PMA is set in the powdered culture medium supply unit 17A, and the powdered culture medium PMB is set in the powdered culture medium supply unit 17B. The powdered culture medium PMA is composed of components (group A) having high solubility in an alkaline solvent, that is, the second initial solvent IS2A, among the components of the powdered culture medium PM. On the other hand, the powdered culture medium PMB is composed of components (group B) having high solubility in an acidic solvent, that is, the second initial solvent IS2B, among the components of the powdered culture medium PM. In the mixing container 16A, a solution SA in which the powdered culture medium PMA is dissolved is prepared. On the other hand, in the mixing container 16B, a solution SB in which the powdered culture medium PMB is dissolved is prepared.

One end of the solution flow passage 65A is connected to the mixing container 16A. In addition, one end of the solution flow passage 65B is connected to the mixing container 16B. The solution SA flows through the solution flow passage 65A from the mixing container 16A. The solution SB flows through the solution flow passage 65B from the mixing container 16B.

The other end of the solution flow passage 65A and the other end of the solution flow passage 65B are connected to the mixer 66. The mixer 66 mixes the solution SA from the solution flow passage 65A and the solution SB from the solution flow passage 65B to produce the culture medium CM. The culture medium supply line 35 is connected to the mixer 66, and the culture medium CM produced by the mixer 66 is supplied to the culture tank 11 through the culture medium supply line 35. The mixer 66 is, for example, a rotary mixer that includes a stirring blade inside and mixes the two liquids by rotating the stirring blade.

As described above, in the fifth embodiment, the powdered culture medium PM is classified into the group A and the group B according to the solubility. Then, the second initial solvent IS2A and the second initial solvent IS2B are prepared for each group, and the solution SA and the solution SB are prepared for each group. Finally, the solution SA and the solution SB prepared for each group are mixed to produce a culture medium CM. Therefore, the time taken for the concentration of the culture medium CM to reach the set concentration can be shortened as compared with a case where the powdered culture medium PM is not classified into groups according to the solubility. As a result, the amount of the culture medium CM to be discarded can be further reduced, and the cost can be further reduced. It is noted that the group is not limited to two groups of the A group and the B group, and may be three or more groups.

### [Examples]

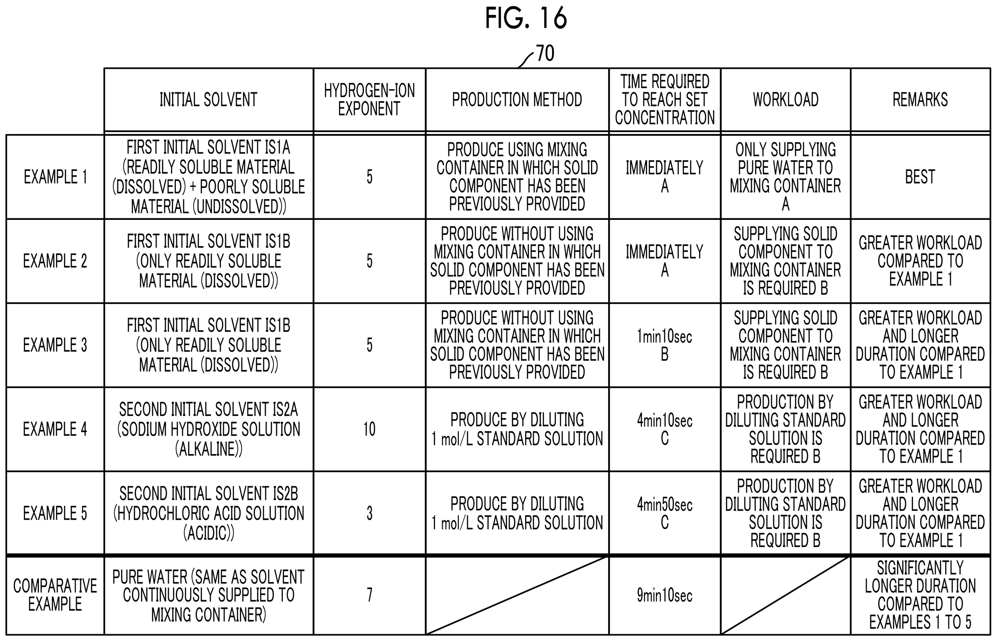

Fig. 16 shows a table 70 summarizing Examples and Comparative Examples. First, the devices used in Examples and Comparative Examples are listed. A beaker (model number: B-200 SCI, capacity: 200 mL) manufactured by HARIO Co., Ltd. was used as the mixing container 16. As the powdered culture medium supply unit 17, a powder weighing and feeding machine (model number: PSF-100SA) manufactured by AS ONE Corporation was used. A stirrer used to crush rat holes in the hopper 30 was Tornado (stirrer) with a timer (model number: SMT-104) manufactured by AS ONE Corporation.

For each flow passage such as the pure water supply line 21, the culture medium supply line 35, and the waste liquid line 42, a lab silicone tube (product number: 9-869-07, inner diameter × outer diameter = φ4 mm × φ6 mm) manufactured by AS ONE Corporation or the like was used after being appropriately cut.

A Masterflex pump (model number: 07528-30) manufactured by Yamato Scientific Co., Ltd. was used as a driving unit of the pure water supply pump 22 and the culture medium supply pump 36. A Masterflex pump head (model number: 77201-60) manufactured by Yamato Scientific Co., Ltd. was used as a pump head of the pure water supply pump 22 and the culture medium supply pump 36. In the driving units of the pure water supply pump 22 and the culture medium supply pump 36, the above-described Masterflex pump was shared for both purposes, and two of the above-described Masterflex pump heads were connected in tandem to one Masterflex pump.

A clamp-on type flow sensor (model number: FD-XA1) manufactured by KEYENCE CORPORATION was used as the pure water flowmeter 23 and the culture medium flowmeter 37.

An Ultra stirrer (model number: MSD-1) manufactured by AS ONE Corporation was used as the stirrer 32. As the stirring bar 33, a regular stirrer bar (model number: C 8 × 30, diameter × length = φ8 mm × 30 mm) manufactured by AS ONE Corporation was used.

As the main bodies of the conductivity meter 39 and the hydrogen-ion exponent meter 40, a benchtop potential hydrogen (pH) and conductivity meter (model number: F-74) manufactured by Horiba, Ltd. was also shared for both purposes. As the electrode of the conductivity meter 39, a general-purpose conductivity cell (model number: 3562-10D) manufactured by HORIBA, Ltd. was used. As an electrode of the hydrogen-ion exponent meter 40, a non-refillable pH electrode (model number: 9652-20D) manufactured by HORIBA, Ltd. was used. In all of these electrodes, the conductivity and the hydrogen-ion exponent can be measured inline. As the flow cell installed together with these electrodes, a flow cell (product number: 3200844642) manufactured by HORIBA, Ltd. was used.

Subsequently, the materials used in Examples and Comparative Examples are listed. As the powdered culture medium PM, a minimum essential medium (MEM) (product number: 41500083) manufactured by Gibco was used. The sodium hydroxide solution used as the second initial solvent IS2A of Example 4 was produced by using a 1 mol/L sodium hydroxide solution (product number: 192-02175) as a standard solution manufactured by FUJIFILM Wako Pure Chemical Corporation and appropriately diluting the solution. The hydrochloric acid solution used as the second initial solvent IS2B of Example 5 was produced by using a 1 mol/L hydrochloric acid solution (product number: 084-03345) as a standard solution manufactured by FUJIFILM Wako Pure Chemical Corporation and appropriately diluting the solution.

Finally, experimental conditions of Examples and Comparative Examples are listed. The flow rate of the pure water PW supplied to the mixing container 16 is 80 mL/min. The supply amount of the powdered culture medium PM to the mixing container 16 per unit time is 0.76 g/min. The rotation speed of the screw of the feeder 31 of the powdered culture medium supply unit 17 is 1 rotation per minute (rpm). The amount of the liquid in the mixing container 16 is 100 mL. The rotation speed of the stirrer 32 is 300 rpm. The measurement interval of the conductivity meter 39 and the hydrogen-ion exponent meter 40 is 10 seconds.

The flow rate of the pure water PW was calculated from the supply amount of the powdered culture medium PM, with a value that matched the "amount of water to be put in with respect to a certain amount of powder" described in the instruction manual of the powdered culture medium PM. As the supply amount of the powdered culture medium PM, a value obtained by actually supplying the powdered culture medium PM by rotating the screw of the feeder 31 at a set rotation speed and measuring the weight of the powdered culture medium PM supplied in a unit time with an electronic balance was used. As the electronic balance, a compact balance (model number: EW-150i) manufactured by A&D Company, Limited was used.

As described above, since the supply amount of the pure water PW to the mixing container 16 and the discharge amount of the culture medium CM from the mixing container 16 was appropriately controlled, the amount of the liquid in the mixing container 16 did not change from the beginning to the end. In a case where the amount of the liquid in the mixing container 16 before the continuous supply of the pure water PW and the powdered culture medium PM was started, that is, the amount of the initial solvent was too large, the time taken for the concentration of the culture medium CM to reach the set concentration was prolonged, or partial bias occurred in the concentration of the culture medium CM in the mixing container 16. On the other hand, in a case where the amount of the initial solvent in the mixing container 16 before the continuous supply of the pure water PW and the powdered culture medium PM was started was too small, the supply amount of the powdered culture medium PM became too large with respect to the amount of the initial solvent, and poor dissolution tended to occur, for example, in a case where the powdered culture medium PM accumulated on the liquid surface. In consideration of this, the amount of the liquid in the mixing container 16 was set to 100 mL, which was neither too much nor too little with respect to the supply amount of the powdered culture medium PM.

The hydrogen-ion exponents shown in Table 70 are the hydrogen-ion exponents of the initial solvents. The time taken to reach the set concentration is a settling time in a case where an error range is ±5% from the set concentration (set value of the conductivity). In the evaluation of the time taken to reach the set concentration, with respect to 9 minutes and 10 seconds of Comparative Example, a case where the time was more than 20% and 60% or less was evaluated as C, a case where the time was more than 5% and 20% or less was evaluated as B, and a case where the time was 5% or less was evaluated as A. The evaluation of the workload is a relative evaluation in a case where Example 1, which is considered to have the least workload, is denoted as A.

Example 1 is a case where the initial solvent is the first initial solvent IS1A of the first embodiment, and a case where the first initial solvent IS1A is produced using the mixing container 16 in which the solid components SC has been previously provided by the method shown in Fig. 3 or Fig. 6. The time taken to reach the set concentration is immediate, and the evaluation is A. Since the workload of the operator is only to supply the pure water PW to the mixing container 16, the evaluation is A. Therefore, it is the best among Examples.

Example 2 is a case where the first initial solvent IS1A is produced by the method shown in Fig. 7 or Fig. 8 without using the mixing container 16 in which the solid components SC has been previously provided, although the initial solvent is the first initial solvent IS1A of the first embodiment as in Example 1. The time taken to reach the set concentration is immediate as in Example 1, and the evaluation is also A as in Example 1. Provided that since the solid component SC needs to be supplied to the mixing container 16, the evaluation of the workload of the operator is B. That is, the workload is large as compared with Example 1.

Example 3 is a case where the initial solvent is the first initial solvent IS1B of the second embodiment, and a case where the first initial solvent IS1B is produced without using the mixing container 16 in which the solid components SC has been previously provided by the method shown in Fig. 7 or Fig. 8. The time taken to reach the set concentration is 1 minute and 10 seconds, and the evaluation is B. Since the solid component SC needs to be supplied to the mixing container 16, the evaluation of the workload of the operator is B, as in Example 2. That is, the time taken to reach the set concentration is longer and the workload is larger than that in Example 1.

Example 4 is a case where the initial solvent is the second initial solvent IS2A (sodium hydroxide solution) of the third embodiment. The hydrogen-ion exponent is 10 and is in a range of 8.6 to 14. The time taken to reach the set concentration is 4 minutes and 10 seconds, and the evaluation is C. Since the initial solution needs to be produced by diluting the sodium hydroxide solution as a standard solution, the evaluation of the workload is B. That is, the time taken to reach the set concentration is longer and the workload is larger than that in Example 1.

Example 5 is a case where the initial solvent is the second initial solvent IS2B (hydrochloric acid solution) of the fourth embodiment. The hydrogen-ion exponent is 3 and is in a range of 0 or more and 5.8 or less. The time taken to reach the set concentration was 4 minutes and 50 seconds, and the evaluation was C. Since the initial solution needs to be produced by diluting the hydrochloric acid solution as a standard solution, the evaluation of the workload is B. That is, the time taken to reach the set concentration is longer and the workload is larger than that in Example 1. It is considered that in Examples 4 and 5, the effect is more exhibited as the amount of the poorly soluble material having a relatively slow dissolution rate is larger.

In Comparative Examples, the initial solvent was pure water PW in the related art. The time taken to reach the set concentration is 9 minutes and 10 seconds. That is, the time taken to reach the set concentration is extremely long as compared with Example 1-5.

From the above, it was confirmed that in Examples 1 to 5 (first embodiment to fourth embodiment) according to the technique of the present disclosure, the time taken for the concentration of the culture medium CM to reach the set concentration can be significantly shorter than that in Comparative Example, and thus the cost can be reduced as compared with the related art. In particular, it was found that Example 1 was the best in terms of both the time taken to reach the set concentration and the workload.

The conductivity measured by the conductivity meter 39 is a representative value obtained by summing the conductivities of the respective components dissolved in the culture medium CM. Therefore, to more accurately determine whether or not the concentration of the culture medium CM has reached the set concentration, it is preferable to measure the concentration of each component of the culture medium CM. However, since it is not easy to measure the concentration of each component of the culture medium CM, a representative value of the conductivity of each component is measured instead.

In a case where the concentration of each component of the culture medium CM can be measured, it is best to determine, based on the result, whether or not the concentration of the culture medium CM has reached the set concentration. Provided that in a case where the concentration of each component of the culture medium CM is measured, there is a possibility that the time taken for each component to reach the set concentration may vary. Therefore, in a case where it is determined that all the components have reached the set concentration, it is necessary to stop discarding the culture medium CM and start supplying the culture medium CM to the culture tank 11.

The characteristics of the culture medium CM to be supplied to the culture tank 11, which are to be measured inline, are not limited to the exemplified conductivity and hydrogen-ion exponent. In addition to or instead of these, a Raman spectrum, an infrared absorption spectrum, a near infrared absorption spectrum, an ultraviolet absorption spectrum, a fluorescence spectrum, and the like may be measured.

The method of measuring the timing at which the discard of the culture medium CM is stopped and the supply of the culture medium CM to the culture tank 11 is started is not limited to the method based on the conductivity in each of the embodiments. The time taken for the concentration of the culture medium CM to reach the set concentration may be measured and stored in advance. Then, in a case where the elapsed time from the start of the continuous supply of the pure water PW and the powdered culture medium PM to the mixing container 16 is timed and it reaches the stored time, the discard of the culture medium CM may be stopped and the supply of the culture medium CM to the culture tank 11 may be started. In this case, the conductivity meter 39 is not necessary.

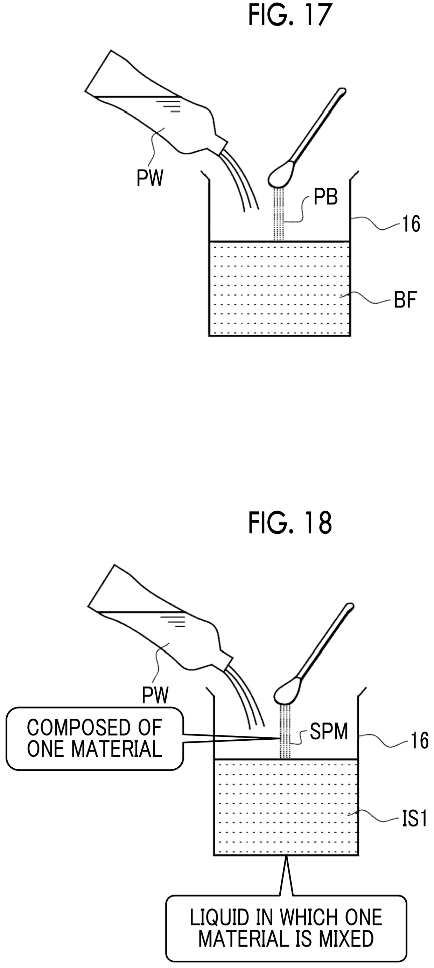

In each of the embodiments, pure water PW is exemplified as the solvent and the purified water, but the present invention is not limited thereto. Distilled water or the like may be used. In addition, in each of the embodiments, the culture medium CM is exemplified as the bioprocess solution, but the present invention is not limited thereto. As shown in Fig. 17 as an example, the bioprocess solution may be a buffer solution BF. Therefore, the solid bioprocess material is not limited to the exemplified powdered culture medium PM, and may be the powder PB constituting the solid component of the buffer solution BF.

In addition, the solid process material may not be composed of a plurality of materials, such as the powdered culture medium PM of each of the embodiments. Similarly, the first initial solvent IS1 may not be a liquid in which a plurality of materials are mixed as in each of the embodiments. As shown in Fig. 18 as an example, the solid process material SPM may be composed of only a single material, and the first initial solvent IS1 may be a liquid in which the one material is mixed. In this case, the single material may be any of a readily soluble material or a poorly soluble material. In a case where the single material is a readily soluble material, the single material is present in a state of being dissolved in the first initial solvent IS1. In a case where the single material is a poorly soluble material, the single material is present in a state where a certain amount of the first initial solvent IS1 is accumulated. As described above, the present invention provides the effect that even in a case where the solid bioprocess material SPM is composed of only a single material and the first initial solvent IS1 is a liquid in which the single material is mixed, it is possible to reduce the amount of the bioprocess solution to be discarded, and it is possible to reduce the cost.

As shown in Fig. 18, in a case where the solid process material SPM is composed of only a single material and the first initial solvent IS1 is a liquid in which the single material is mixed, the first initial solvent IS1 is produced by using the mixing container 16. For example, the first initial solvent IS1 is produced by supplying a solvent to a mixing container 16 in which the solid component SC of the first initial solvent IS1 has been previously provided. In this case, the solvent is purified water, the solid bioprocess material SPM is the powdered culture medium PM, and the bioprocess solution is the culture medium CM. Then, the culture medium CM is continuously supplied from the mixing container 16 to the culture tank 11. Alternatively, the solvent may be purified water, the solid bioprocess material SPM may be a powder PB constituting a solid component of the buffer solution BF, and the bioprocess solution may be the buffer solution BF. Furthermore, in this case, it is preferable that the mixing container 16 is disposable after one use.

The expression "the solid process material SPM is composed of only a single material" means that the solid process material SPM is composed of only a single compound. However, the present invention also includes a case where impurities are mixed in an amount that is generally allowed in the technical field to which the technology of the present disclosure belongs and does not contradict the gist of the technology of the present disclosure, in addition to the single compound.

The technology according to the following appendices can be perceived from the above description.

### [Appendix 1]

A method for preparing a bioprocess solution, in which a bioprocess solution having a set concentration is continuously prepared in a mixing container by continuously supplying a solvent and a solid bioprocess material to the mixing container, the method comprising:
storing a first initial solvent, which has a composition closer to the bioprocess solution having the set concentration than the solvent continuously supplied to the mixing container, or a second initial solvent, which has a higher solubility for the solid bioprocess material than the solvent continuously supplied to the mixing container, in the mixing container; and
starting continuous supply of the solid bioprocess material and the solvent to the mixing container in a state where the first initial solvent or the second initial solvent is stored in the mixing container.

### [Appendix 2]

The method for preparing a bioprocess solution according to appendix 1,
wherein the solid bioprocess material includes a poorly soluble material having a relatively slow dissolution rate in the solvent, which remains undissolved and accumulates in the mixing container for a certain period of time after being supplied to the mixing container, and whose solubility increases in a case where a certain amount has accumulated, and
the first initial solvent is a liquid in which the poorly soluble material is not dissolved.

### [Appendix 3]

The method for preparing a bioprocess solution according to appendix 2,
wherein the first initial solvent contains a set amount of the poorly soluble material based on the certain amount.

### [Appendix 4]

The method for preparing a bioprocess solution according to any one of appendixes 1 to 3,
wherein the first initial solvent is produced by using the mixing container.

### [Appendix 5]

The method for preparing a bioprocess solution according to appendix 4,
wherein the first initial solvent is produced by supplying the solvent to the mixing container in which a solid component of the first initial solvent has been previously provided.

### [Appendix 6]

The method for preparing a bioprocess solution according to appendix 1,
wherein an alkaline liquid is used as the second initial solvent.

### [Appendix 7]

The method for preparing a bioprocess solution according to appendix 6,
wherein a liquid having a hydrogen-ion exponent of 8.6 or more and 14 or less is used as the second initial solvent.

### [Appendix 8]

The method for preparing a bioprocess solution according to appendix 1,
wherein an acidic liquid is used as the second initial solvent.

### [Appendix 9]

The method for preparing a bioprocess solution according to appendix 8,
wherein a liquid having a hydrogen-ion exponent of 0 or more and 5.8 or less is used as the second initial solvent.

### [Appendix 10]

The method for preparing a bioprocess solution according to any one of appendixes 1 and 6 to 9,
wherein the solid bioprocess material is divided into a plurality of groups according to solubility,
the second initial solvent is provided for each of the groups to prepare solutions for each of the groups, and
the solutions prepared for each of the groups are mixed to produce the bioprocess solution.

### [Appendix 11]

The method for preparing a bioprocess solution according to any one of appendixes 1 to 10,
wherein the solvent is purified water, the solid bioprocess material is a powdered culture medium, and the bioprocess solution is a culture medium.

### [Appendix 12]

The method for preparing a bioprocess solution according to appendix 11,
wherein the culture medium is continuously supplied from the mixing container to a culture tank.

### [Appendix 13]

The method for preparing a bioprocess solution according to any one of appendixes 1 to 10,
wherein the solvent is purified water, the solid bioprocess material is a powder constituting a solid component of a buffer solution, and the bioprocess solution is a buffer solution.

### [Appendix 14]

The preparation method of a bioprocess solution according to any one of appendixes 1 to 13,
wherein the mixing container is disposable after one use.

### [Appendix 15]

The method for preparing a bioprocess solution according to any one of appendixes 1 to 14,
wherein the solid bioprocess material is composed of only a single material, and
the first initial solvent is a liquid in which the single material is mixed.

### [Appendix 16]

A mixing container in which a bioprocess solution having a set concentration is continuously prepared by continuously supplying a solvent and a solid bioprocess material,
wherein an initial solvent is stored before the continuous supply of the solid bioprocess material and the solvent is started,
the initial solvent includes a solid component that is previously provided and has a composition closer to a composition of the bioprocess solution at the set concentration than a composition of the continuously supplied solvent, and
the initial solvent is produced by supplying the solvent.

The technology of the present disclosure can also be combined with various embodiments and/or various modification examples described above, as appropriate. In addition, it goes without saying that the present disclosure is not limited to each of the embodiments described above, various configurations can be adopted as long as the configuration does not deviate from the gist.

The above-described contents and the above-shown contents are the detailed description of the parts according to the technology of the present disclosure, and are merely an example of the technology of the present disclosure. For example, the above description of the configuration, the function, the operation, and the effect are the description of examples of the configuration, the function, the operation, and the effect of the parts according to the technology of the present disclosure. Accordingly, it goes without saying that unnecessary parts may be deleted, new elements may be added, or replacements may be made with respect to the above-described contents and the above-shown contents within a range that does not deviate from the gist of the technology of the present disclosure. In addition, in order to avoid complications and facilitate grasping the parts according to the technology of the present disclosure, in the above-described contents and the above-shown contents, the description of technical general knowledge and the like that do not particularly require description for enabling the implementation of the technology of the present disclosure are omitted.

In the present specification, "A and/or B" has the same meaning as "at least one of A or B". That is, "A and/or B" means that it may be only A, only B, or a combination of A and B. In addition, in the present specification, also in a case in which three or more matters are expressed in association by "and/or", the same concept as "A and/or B" is applied.

All of the documents, the patent applications, and the technical standards described in the present specification are incorporated herein by reference to the same extent as in a case in which each of the documents, patent applications, and technical standards is specifically and individually described by being incorporated by reference.

## Claims

1. A method for preparing a bioprocess solution, in which a bioprocess solution having a set concentration is continuously prepared in a mixing container by continuously supplying a solvent and a solid bioprocess material to the mixing container, the method comprising:
storing a first initial solvent, which has a composition closer to the bioprocess solution having the set concentration than the solvent continuously supplied to the mixing container, or a second initial solvent, which has a higher solubility for the solid bioprocess material than the solvent continuously supplied to the mixing container, in the mixing container; and
starting continuous supply of the solid bioprocess material and the solvent to the mixing container in a state where the first initial solvent or the second initial solvent is stored in the mixing container.

2. The method for preparing a bioprocess solution according to claim 1,
wherein the solid bioprocess material includes a poorly soluble material having a relatively slow dissolution rate in the solvent, which remains undissolved and accumulates in the mixing container for a certain period of time after being supplied to the mixing container, and whose solubility increases in a case where a certain amount has accumulated, and
the first initial solvent is a liquid in which the poorly soluble material is not dissolved.

3. The method for preparing a bioprocess solution according to claim 2,
wherein the first initial solvent contains a set amount of the poorly soluble material based on the certain amount.

4. The method for preparing a bioprocess solution according to claim 1,
wherein the first initial solvent is produced by using the mixing container.

5. The method for preparing a bioprocess solution according to claim 4,
wherein the first initial solvent is produced by supplying the solvent to the mixing container in which a solid component of the first initial solvent has been previously provided.

6. The method for preparing a bioprocess solution according to claim 1,
wherein an alkaline liquid is used as the second initial solvent.

7. The method for preparing a bioprocess solution according to claim 6,
wherein a liquid having a hydrogen-ion exponent of 8.6 or more and 14 or less is used as the second initial solvent.

8. The method for preparing a bioprocess solution according to claim 1,
wherein an acidic liquid is used as the second initial solvent.

9. The method for preparing a bioprocess solution according to claim 8,
wherein a liquid having a hydrogen-ion exponent of 0 or more and 5.8 or less is used as the second initial solvent.

10. The method for preparing a bioprocess solution according to claim 1,
wherein the solid bioprocess material is divided into a plurality of groups according to solubility,
the second initial solvent is provided for each of the groups to prepare solutions for each of the groups, and
the solutions prepared for each of the groups are mixed to produce the bioprocess solution.

11. The method for preparing a bioprocess solution according to claim 1,
wherein the solvent is purified water, the solid bioprocess material is a powdered culture medium, and the bioprocess solution is a culture medium.

12. The method for preparing a bioprocess solution according to claim 11,
wherein the culture medium is continuously supplied from the mixing container to a culture tank.

13. The method for preparing a bioprocess solution according to claim 1,
wherein the solvent is purified water, the solid bioprocess material is a powder constituting a solid component of a buffer solution, and the bioprocess solution is a buffer solution.

14. The preparation method of a bioprocess solution according to claim 1,
wherein the mixing container is disposable after one use.

15. The method for preparing a bioprocess solution according to claim 1,
wherein the solid bioprocess material is composed of only a single material, and
the first initial solvent is a liquid in which the single material is mixed.

16. A mixing container in which a bioprocess solution having a set concentration is continuously prepared by continuously supplying a solvent and a solid bioprocess material,
wherein an initial solvent is stored before the continuous supply of the solid bioprocess material and the solvent is started,
the initial solvent includes a solid component that is previously provided and has a composition closer to a composition of the bioprocess solution at the set concentration than a composition of the continuously supplied solvent, and
the initial solvent is produced by supplying the solvent.
